# EUROPEAN PATENT SPECIFICATION

(11) **EP 3 460 052 B1**
(45) Date of publication and mention of the grant of the patent: **30.10.2019**
(21) Application number: 17192226.3
(22) Date of filing: 20.09.2017
(51) Int. Cl.: C12N 5/0784, A61K 35/15, C12N 15/861

(54) **IMPROVED ALLOGENEIC DENDRITIC CELLS FOR USE IN CANCER TREATMENT**
VERBESSERTE ALLOGENE DENDRITISCHE ZELLEN ZUR VERWENDUNG IN DER KREBSBEHANDLUNG
CELLULES DENDRITIQUES ALLOGÉNIQUES AMÉLIORÉES DESTINÉES À ÊTRE UTILISÉES DANS LE TRAITEMENT DU CANCER

(43) Date of publication of application: 27.03.2019
(73) Proprietor: Immunicum AB, 114 42 Stockholm (SE)
(72) Inventor: KARLSSON-PARRA, Alex, 141 68 Huddinge (SE)
(74) Representative: Ström & Gulliksson AB

(56) References cited:
- SHIJIE HU ET AL: "Induction of antigen-specific cytotoxic T-cell response by dendritic cells generated from ecto-mesenchymal stem cells infected with an adenovirus containing the MAGE-D4a gene", ONCOLOGY LETTERS, 7 March 2016 (2016-03-07), XP055430933, GR ISSN: 1792-1074, DOI: 10.3892/ol.2016.4306
- JAMES M. TERMINI ET AL: "Epstein Barr virus Latent Membrane Protein-1 enhances dendritic cell therapy lymph node migration, activation, and IL-12 secretion", PLOS ONE, vol. 12, no. 9, 14 September 2017 (2017-09-14), page e0184915, XP055430943, DOI: 10.1371/journal.pone.0184915
- YI ZHENG ET AL: "Dendritic cells infected by Ad-sh-SOCS1 enhance cytokine-induced killer (CIK) cell immunotherapeutic efficacy in cervical cancer models", CYTOTHERAPY, vol. 19, no. 5, 1 May 2017 (2017-05-01), pages 617-628, XP055430945, GB ISSN: 1465-3249, DOI: 10.1016/j.jcyt.2017.01.008
- KATY R. NEWTON ET AL: "Human dendritic cells infected with an adenoviral vector suppress proliferation of autologous and allogeneic T cells", IMMUNOLOGY, vol. 125, no. 4, 1 December 2008 (2008-12-01), pages 469-479, XP055430947, GB ISSN: 0019-2805, DOI: 10.1111/j.1365-2567.2008.02860.x
- LÖVGREN TANJA ET AL: "Enhanced stimulation of human tumor-specific T cells by dendritic cells matured in the presence of interferon-[gamma] and multiple toll-like receptor agonists", CANCER IMMUNOLOGY, IMMUNOTHERAPY, SPRINGER, BERLIN/HEIDELBERG, vol. 66, no. 10, 10 June 2017 (2017-06-10) , pages 1333-1344, XP036330841, ISSN: 0340-7004, DOI: 10.1007/S00262-017-2029-4 [retrieved on 2017-06-10]

## Description

### FIELD OF THE INVENTION

The present invention relates to a method of providing pro-inflammatory dendritic cells with ability to activate allogeneic T-cells through the direct pathway of allorecognition, as well as to the use of such pro-inflammatory dendritic cells in cancer treatment.

### BACKGROUND

Traditional cancer therapies, such as surgery, radiation, and chemotherapy, are often insufficient in treating cancer patients and usually cause severe side effects. Immunotherapy has shown promise as an alternative treatment method with less negative side effects.

Cancer immunotherapy has made enormous progress recently thanks to the introduction of checkpoint blockade antibodies, in particular antibodies blocking PD-1 (programmed cell death protein 1) and PD-1 ligand (PD-L1) interactions. However, only a fraction of cancer patients benefits from this kind of treatment. Further, it is only effective against certain forms of cancer. As there is still room for improvement in the field, cancer immunotherapies evolve to be more efficient and robust.

It is now well established that the immune system has cells, particularly CD8+ cytotoxic T lymphocytes (CTLs), which can recognize and potentially kill tumor cells. Nevertheless, a major problem is that the killing ability of these T cells are either not induced or only weakly induced in cancer patients. One possibility is that there is inadequate tumor antigen presentation and co-stimulation by dendritic cells (DCs) for eliciting a functional and tumor-specific T cell immunity in cancer patients. Thus, it is of interest to develop immunotherapy strategies for "turning-on" the immune system to have it recognizing and killing tumor cells.

Existing cancer immunotherapy strategies, involving activation of CD8+ cytotoxic T lymphocytes (CTLs), have mainly focused on antigen-loaded autologous, patient-derived DCs, which have been differentiated and antigen-loaded *ex vivo.* The underlying premise of this approach is that the efficiency and control provided by *ex vivo* manipulation of the DCs generates DCs with strong antigen-presenting and co-stimulatory capacity. The quality of the T cell response depends on the ability of these autologous DCs to present tumor antigens to T cells in a MHC-restricted manner (DCs and T cells have to be from the same individual, i.e. autologous) in draining lymph nodes and thus create a tumor-specific T cell response.

The patient-derived, *ex vivo*-modified dendritic cells (DCs) aim to induce tumor-specific T-cell immunity by direct education of T-cells against tumor-associated, or tumor-specific, antigens. Although proof-of-principle has been observed for autologous DCs vaccines in several clinical studies, clinical responses are yet suboptimal (Anguille, S., et al. 2014. Clinical use of dendritic cells for cancer therapy. Lancet Oncol 15: e257-267). Further, variable clinical outcomes seem to correlate with the activation status of the vaccine DCs, thus consensus in the field at the moment drives optimization and standardization processes for the production of immune enhancers capable to induce DC-mediated T-helper type-1 (Th1)-polarized immune responses.

Interestingly though, recent findings indicate that *ex vivo*-modified vaccine DCs actually requires help from endogenous bystander-DCs to prime CD8+ T-cell specific immunity (Yewdall, A. W., et al 2010. CD8+ T cell priming by dendritic cell vaccines requires antigen transfer to endogenous antigen presenting cells. PLoS One 5: e11144; Liu, C., et al 2008. Plasmacytoid dendritic cells induce NK cell-dependent, tumor antigen-specific T cell cross-priming and tumor regression in mice. J Clin Invest 118: 1165-1175). This indirect priming of bystander-DCs has been found by many to be dependent on the active secretion of immune cell-recruiting and immune cell-activating pro-inflammatory factors by the administered DCs.

Immature monocyte-derived human DCs can secrete substantial amounts of T-helper (Th)-1 cytokines and chemokines when stimulated by a cocktail of stimuli, such as Toll-like receptor (TLR) ligands and IFN-γ (Napolitani, G., A. et al 2005. Selected Toll-like receptor agonist combinations synergistically trigger a T helper type 1-polarizing program in dendritic cells. Nat Immunol 6: 769-776; Lovgren, T., D. et al 2017. Enhanced stimulation of human tumor-specific T cells by dendritic cells matured in the presence of interferon-gamma and multiple toll-like receptor agonists. Cancer Immunol Immunother.). Further, the role the indirect priming of endogenous bystander-DCs implies that the local administration of allogeneic DCs (alloDCs) can create an enriched milieu of pro-inflammatory factors through the so called "direct pathway of allorecognition", subsequently leading to enhanced recruitment and activation of endogenous bystander DCs by pro-inflammatory factors released by recruited and activated alloreactive T cells (Wallgren, A. C et al 2005. Direct allorecognition promotes activation of bystander dendritic cells and licenses them for Th1 priming: a functional link between direct and indirect allosensitization. Scand J Immunol 62: 234-242). This direct pathway of allorecognition, a phenomenon where up 10 % of the T cells from the transplanted recipient can become activated by their direct recognition of intact allogeneic MHC/peptide complexes present on graft-derived allogeneic DCs (cf. Lombardi G, et al Allorecognition of DR1 by T cells from a DR4/DRw13 responder mimics selfrestricted recognition of endogenous peptides. Proc Natl Acad Sci USA 1989;86:4190-4), is a vigorous response, which appears to violate the rule of self-MHC restriction, and is primarily driven by antigenic mimicry (cf. Lechler R, et al Dendritic cells in transplantation - friend or foe? Immunity 2001;14:357-68).

The use of allogeneic DCs (alloDCs) has been described previously in the art (cf. EP 1 509 244 B1 and EP 2 534 242 B1). Further, the alloDC approach using intratumorally injected pro-inflammatory alloDCs has been tested successfully in a phase I/II clinical trial in patients with metastatic renal cell carcinoma (mRCC) as reported by Laurell, A et al (cf. Intratumorally injected pro-inflammatory allogeneic dendritic cells as immune enhancers: a first-in-human study in unfavourable risk patients with metastatic renal cell carcinoma. Journal for ImmunoTherapy of Cancer, 2017, 5: 52) and is currently subject to further clinical studies in in a randomized multicenter phase II study in mRCC diagnosed patients (NCT02432846). Data from the phase I/II clinical trial in mRCC patients indicated that intratumoral administration of alloDCs induced activation of tumor-specific T cells with strong/massive intratumoral infiltration of CD8+ T cells in the majority of treated patients. Further, a synergistic antitumor effect was seen in combination with sunitinib. It is hypothesized that the alloDCs elicit a tumor-specific T cell response and that the addition of sunitinib makes the tumor more susceptible to the tumor-specific T cell response, as sunitinib has been shown to affect and reduce the number of myeloid-derived suppressor cells and regulatory T cells.

Though, the use of alloDCs in the art seemingly provides a promising immunotherapy strategy, it would still be of interest to be able to further improve the ability of alloDCs in improving the number and function of tumor-specific CD8+ cytotoxic T lymphocytes affecting tumor cells.

### SUMMARY

Consequently, the present invention seeks to mitigate, alleviate, eliminate or circumvent one or more of the above-identified potential deficiencies in the art and disadvantages singly or in any combination by providing, according to a first aspect of the invention, a method of providing a pro-inflammatory dendritic cell having improved ability to activate allogeneic T-cells through the direct pathway of allorecognition and being infected with an adenovirus. Such a method comprises the steps of:
- providing an immature dendritic cell;
- infecting the dendritic cell with an adenovirus; and
- maturating the immature dendritic cell into a pro-inflammatory dendritic cell, wherein said maturation is performed by addition of the Toll-like receptor 3 (TLR3)-ligand poly-I:C, a TLR7/8-ligand, such as Resiquimod, and interferon gamma (IFN-γ) to induce maturation of the immature dendritic cell.

Pro-inflammatory dendritic cells being infected with an adenovirus and matured by the addition of the Toll-like receptor 3 (TLR3)-ligand poly-I:C, a TLR7/8-ligand, such as Resiquimod, and interferon gamma (IFN-γ), has, in contrast to the prevailing opinion, improved ability to stimulate the direct pathway of allorecognition of allogeneic T cells, as determined in vitro during a mixed leukocyte reaction (MLR). Importantly, such pro-inflammatory dendritic cells thereby also have increased ability to eventually, via activation/maturation of antigen-loaded bystander DCs, increase the number and function of autologous (with respect to the bystander DCs) CD8+ cytotoxic T lymphocytes being able to specifically recognize tumor cells. Thus, these adenovirus-infected pro-inflammatory dendritic cells are useful as an improved cell-based allogeneic immune enhancer in treating or preventing cancer.

Further advantageous features of the invention are defined in the dependent claims. In addition, advantageous features of the invention are elaborated in embodiments disclosed herein.

### Detailed description of preferred embodiments

In immunotherapy based on patient-derived, i.e. autologous, dendritic cells (DCs) are typically modified *ex vivo* to express or comprise tumor antigens (tumor-associated or tumor-specific antigens). As recognized in the art, various means are available for modifying DCs to express or comprise tumor antigens. These means include infection with viral vectors, e.g. adenovirus, adeno-associated virus, lentivirus and retrovirus vectors, with genes coding for tumor antigens.

As already described herein above, also pro-inflammatory allogeneic DCs may be used in immunotherapy, as they have been found to activate bystander DCs. Allogeneic DCs administered intratumorally does not need to be antigen loaded (i.e. express or comprise tumor antigens), as tumor antigens for processing by activated endogenous bystander DCs anyhow is present at the site of administration. The use of allogeneic DCs as cell-based immune enhancers in cancer immunotherapy relies on stimulation of the direct pathway of allorecognition by allogeneic T cells in a mixed leukocyte reaction (MLR), resulting in additional production of pro-inflammatory factors (Wallgren et al, 2005, Scand J Immunol 62: 234-242).

It has previously been shown (cf. EP 2 534 242 B1) that allogeneic DCs, matured into pro-inflammatory DCs by use of a combination of the Toll-like receptor 3 (TLR3)-ligand poly-I:C, Resiquimod (a TLR7/8-ligand), and interferon gamma (IFN-γ) provide a composition useful in intratumoral treatment of cancer, e.g. metastatic renal cell carcinoma.

In evaluating various means for providing DCs carrying tumor antigens, it was, as expected, confirmed that infecting immature DCs with an adenovirus does not impair the ability of a mixture, comprising the Toll-like receptor 3 (TLR3)-ligand poly-I:C, Resiquimod (a TLR7/8-ligand), and interferon gamma (IFN-γ), to induce maturation of the immature DCs into adenovirus-infected pro-inflammatory DCs. The adenovirus-infected pro-inflammatory DCs secreted inflammatory factors in amount corresponding to the one of non-infected, pro-inflammatory DCs, thus confirming the previous reports.

They were however deemed to be of no or little use in an allogeneic setting, given that infection with adenovirus is known in the art to impair the ability of infected DCs to activate allogeneic T cells in a MLR.

Especially, back in 2002, Jonleit *et al* (cf. Jonuleit et al in Gene Therapy (2000) 7, 249-254) reported that infection of DCs with an E1-substituted, E3-deleted adenovirus and maturation with pro-inflammatory factors impaired the ability of the resulting DCs in stimulating the proliferation of allogeneic T cells (CD4+ and CD8+) at high DC to T cell ratio. Similarly, Tan *et al* have reported (cf. BLOOD, (2005), 105(10), 3824- 3832) that infection of matured (TNF-alpha, IL-1 beta, IL-6, and PGE2) monocyte-derived DCs with an adenovirus suppressed the ability of the DCs to activate allogeneic T cells in a MLR. Further, Newton *et al* have reported (cf. Immunology (2008), 125, 469-479) that adenovirus-infected DCs matured with pro-inflammatory factors (TNF-alpha, IL-1 beta, IL-6 and PGE2) have a greatly reduced ability to promote proliferation of allogeneic T cells. According to Newton, the effect is specific for adenoviral infection, as the same effect not was seen with mock-infected or lentivirus-infected DCs.

Never the less, the ability of adenovirus-infected pro-inflammatory DCs to activate allogeneic T cells in a MLR was evaluated. Very surprisingly it was found, in deep contrast to prevailing opinion, that adenoviral infection did not impair the ability to activate allogeneic T cells in a MLR, provided that the DCs had been matured using a combination of the Toll-like receptor 3 (TLR3)-ligand poly-I:C, a TLR7/8-ligand, such as Resiquimod, and interferon gamma (IFN-γ).

Actually, it was even found that the adenovirus-infected pro-inflammatory DCs had improved ability to stimulate the direct pathway of allorecognition as determined by the activation of co-cultured allogeneic T cells in a MLR and the increased production of IFN-gamma. Furthermore, not only had the adenovirus-infected pro-inflammatory DCs improved ability to stimulate the direct pathway of allorecognition, but they also provided increased maturation of allogeneic bystander DCs that subsequently led to an enhance ability of these bystander DCs to activate autologous (with respect to bystander DCs) antigen-specific T cells. Thus, adenovirus-infected pro-inflammatory DCs are useful as an improved cell-based allogeneic immune enhancer for use in cancer treatment, in which the mode of action is dependent on the direct pathway of allorecognition.

Accordingly, an embodiment of the present invention relates to a pro-inflammatory dendritic cell infected with an adenovirus. The adenovirus-infected pro-inflammatory dendritic cell has improved ability to activate allogeneic T-cells through the direct pathway of allorecognition. Further, the adenovirus-infected pro-inflammatory dendritic cell has been obtained by:
- providing an immature dendritic cell;
- transducing the dendritic cell with an adenovirus vector; and
- maturating the immature dendritic cell into a pro-inflammatory dendritic cell.
The maturation of the immature dendritic cell into a pro-inflammatory dendritic cell is performed by addition of the Toll-like receptor 3 (TLR3)-ligand poly-I:C, a TLR7/8-ligand, such as Resiquimod, and interferon gamma (IFN-γ) to induce maturation of the immature dendritic cell.

The present maturation cocktail comprising a combination of the Toll-like receptor 3 (TLR3)-ligand poly-I:C, Resiquimod (a TLR7/8-ligand), and interferon gamma (IFN-γ) has been described in WO 2011/098516. The Toll-like receptor 3 (TLR3)-ligand poly-I:C is a synthetic analog of dsRNA comprising a strand of poly(I) annealed to a strand of poly(C). The size of the strand may vary. The size may be 200 base pairs to 8 000 base pairs, such 200 to 1 500 or 1 500 to 8 000 base pairs. The TLR7/8-ligand R848 is also denoted Resiquimod in the art. As an alternative to Resiquimod, Gardiquimod or Imiquimod may be used as TLR7/8-ligands. Typically, the immature DCs are exposed to the activation factors for 8 to 24 hours, such as 18 hours.

According to an embodiment, the maturation factors used are Toll-like receptor 3 (TLR3)-ligand poly-I:C, Resiquimod (a TLR7/8-ligand), and interferon gamma (IFN-γ).

Though not necessary, the activation may, according to alternative embodiment, further include the addition of at least one substance selected from the group consisting of TLR2-ligands, TLR4-ligands, such as bacterial lipopolysaccharide and monophosphoryl lipid A, TLR9-ligands, such as CpG oligonucleotides (ODN) sequences that distinguish microbial DNA from mammalian DNA, Interferon alpha (IFN-α), interleukin 1β (IL-1β), and tumor necrosis factor alpha (TNF-α). Further, the activation does preferably not comprise addition of prostaglandin E2 (PGE2) in order to prevent the mature DCs from becoming migratory DCs that rapidly will leave the injection site (tumor), which would be disadvantageous within the context of this invention.

Subsequent to the activation, the resulting adenovirus-infected pro-inflammatory DCs may be washed to remove essentially all of the activation factors. Thus, the activation factors typically are washed away prior to use of the adenovirus-infected pro-inflammatory DCs as immune enhancer in cancer immune therapy. Removal of the activation factors avoids co-administration of activating factors (added to induce pro-inflammatory DCs *ex vivo*). Co-administration of activation factors most likely will lead to a strong and persistent activation also of intratumorally recruited immature DCs, leading to their differentiation into pro-inflammatory mature DCs rather than the desired differentiation into migratory mature DCs.

In order to improve the ability to activate allogeneic T-cells through the direct pathway of allorecognition, as determined by the mixed leukocyte reaction (MLR), the DC is infected with an adenovirus.

Various adenoviruses are known in the art. In US 9,017,672 a preferred type of adenovirus, being a hexon TAT(transactivator of transcription)-PTD(transduction domain) modified adenovirus with enhanced gene delivery efficiency, is disclosed. This vector has further been studied by Yu, D., as reported 2013 in PLoS One 8: e54952.

According to an embodiment, the present adenovirus is an adenovirus serotype-5 (Ad5). The adenovirus serotype-5 (Ad5), may be an adenovirus serotype-5 (Ad5) with fiber shaft and/or knob from adenovirus serotype-35 (Ad35) in place of the Ad5 fiber shaft and/or knob. As recognized by the skilled person, several serotypes of adenovirus, including the Ad5 and Ad35, are known in the art. By replacing the Ad5 fiber shaft and/or knob with a fiber shaft and/or knob from Ad35 the transduction efficiency is improved. Further, it is preferred if the hexon protein of the adenovirus vector is modified to comprise at least one protein transduction domain (PTD) of the transactivator of transcription (TAT) from human immunodeficiency virus (HIV). By modifying the hexon protein of the adenovirus vector to comprise at least one protein transduction domain (PTD) of the transactivator of transcription (TAT) from human immunodeficiency virus (HIV), the transduction efficiency is improved. The TAT-PTD sequence may be introduced into the hyper variable region 5 (HV5R) of the hexone protein. According to an embodiment, the hexon protein of the adenovirus vector thus comprises an amino acid sequence according to SEQ ID No. 1 (YGRKKRRQRRR). Further, the amino acid sequence according to SEQ ID No. 1 may be flanked by short linkers and may thus comprise an amino acid sequence according to SEQ ID No. 2 (AGGGAGGGYGRKKRRQRRRGGGAGGGA). An example of a preferred adenovirus (Ad5PTDf35) is disclosed in PLoS One (2013) 8(1): e54952. Further, the adenovirus may be an E1-deleted adenovirus. As recognized by the skilled person, E1-deletion implies that the adenovirus is replication-deficient. In order to have better control of the virus within the subject to whom it is administered, it is preferred that the adenovirus is replication-deficient. According to an embodiment, the adenovirus is thus replication-deficient, e.g. E1-deleted. The adenovirus may also be an E3-deleted adenovirus, such as an E1/E3-deleted adenovirus.

Though, the adenoviruses are used in the art as gene delivery vectors, the effects underlying the present invention are not dependent on the inclusion of any gene encoding for a tumor antigen in the adenovirus vector, but on the infection with a adenovirus as such. Thus, the adenovirus-infected pro-inflammatory DC does not, according to an embodiment, comprise any gene encoding for a tumor antigens, i.e. the DCs are infected with the adenovirus, but not transduced with genes coding for tumor antigens.

Although not necessary for allogeneic pro-inflammatory DCs to be administered intratumorally, it may in some applications (when the tumor is difficult to reach by injection or is to small) still be of interest to provide a pro-inflammatory DCs infected with a adenovirus encoding tumor antigens, i.e. infected and transduced DCs, and thus expressing tumor associated or tumor-specific antigens. The adenovirus-infected pro-inflammatory DC may therefore, according to an embodiment, comprise at least one gene encoding for a tumor associated or tumor-specific antigen. By providing a pro-inflammatory DCs infected with an adenovirus comprising at least one gene encoding for a tumor associated or tumor-specific antigen, the infected dendritic cell is transduced to express a tumor associated or tumor specific antigen. Examples of the tumor antigen to be expressed by the transduced pro-inflammatory DC include oncoviral antigens, such as human papillomavirus, Epstein-Barr virus, Kaposis sarcoma virus, or Merkel cell polyoma virus, and mutation-derived neoantigens.

In the providing the present adenovirus-infected pro-inflammatory DCs, immature DCs are to be infected as well as matured into pro-inflammatory DCs. Aspects of the infection as well as the maturation has already been described herein. According to an embodiment, the infection and maturation, respectively, is performed simultaneously. According to an alternative embodiment, the immature DC is firstly infected to provide an immature, adenovirus-infected DC and subsequently matured into an adenovirus-infected pro-inflammatory DC.

Provision of immature DCs is well known in the art. Typically, monocytes are firstly isolated from peripheral blood leukocytes, e.g. by leukapheresis of whole blood. The isolated monocytes may then be differentiated into immature dendritic cells by use of interleukin 4 (IL-4) and Granulocyte-Macrophage Colony-Stimulating Factor (GM-CSF). The monocyte may be cultured in cell culture medium supplemented with granulocyte-macrophage colony stimulating factor (GM-CSF) in combination with interleukin-4 (IL-4), for 2 to 7 days, such as about 5 days, to differentiate the monocytes into immature DCs. Further, in WO 2014/096033 a process for providing immature DCs is disclosed.

By freezing the pro-inflammatory dendritic cells subsequent to the activation, they may be stored. Typically, the pro-inflammatory dendritic cells are frozen in a medium containing dimethylsulphoxide (DMSO) and serum or plasma. Before use, the frozen cells are thawed and the DMSO may be washed away. Alternatively, the thawed cells are used directly.

For use in the treatment of cancer, the adenovirus-infected pro-inflammatory dendritic cell may be formulated into a pharmaceutical composition. The pharmaceutical composition may comprise at least one pharmaceutical acceptable carrier, such as a phosphate buffered saline solution, water, or an emulsion, such as an oil/water or water/oil emulsion. The pharmaceutical composition may further comprise a wetting agent. Further, the pharmaceutical composition may comprise pharmaceutical acceptable adjuvants, excipients, stabilizers preservatives and/or other components known in the art.

As an example, the carrier may be a saline solution comprising human serum albumin. Further, the carrier may be a medium preserving frozen cells. As example, the adenovirus-infected pro-inflammatory dendritic cell may, as already mentioned, be frozen in heat inactivated universal donor plasma comprising dimethyl sulfoxide (DMSO) to allow for storage thereof. Such a medium comprising adenovirus-infected pro-inflammatory dendritic cells may be used directly, e.g. injected intratumorally, once thawed. Alternatively, cells frozen in such a medium may be thawed, washed and resuspended in saline comprising human serum albumin before being administered, e.g. injected intratumorally.

The pharmaceutical acceptable composition may be formulated for parenteral administration, such as intratumoral, intradermal, subcutaneous or intranodal administration. In intratumoral administration, the pharmaceutical acceptable composition is injected directly into a tumor. As already described (cf. WO 2011/098516), pro-inflammatory dendritic cells are useful in the treatment of cancer, as they may induce a pro-inflammatory reaction via a mixed leukocyte reaction (MLR) at the site of administration recruiting and activating a patient's own DCs (herein denoted bystander DCs) to develop into tumor-loaded migratory DCs.

A further embodiment thus relates to adenovirus-infected pro-inflammatory dendritic cell, or such a composition comprising such adenovirus-infected pro-inflammatory dendritic cells for use in the treatment or prevention of cancer. In such use, the adenovirus-infected pro-inflammatory dendritic cells are allogeneic to the subject, in order to be able to induce a MLR activating the subjects own alloreactive T cells, being allogeneic to the administered adenovirus-infected pro-inflammatory dendritic cells. Examples of cancers to be treated by the adenovirus-infected pro-inflammatory dendritic cells, includes, but are not limited to renal cell carcinoma, hepatocellular carcinoma, gastrointestinal stromal tumors, bile-duct cancer, head and neck cancers, non-small cell lung cancer, gastric cancer, cervical cancer and malignant melanoma.

When used in treating cancer, adenovirus-infected pro-inflammatory dendritic cells may be combined with other existing therapies. A preferred alternative is to combine administration of adenovirus-infected pro-inflammatory dendritic cells with drugs decreasing the immunosuppresive tumor environment or activating the immune system. As an example, the present adenovirus-infected pro-inflammatory dendritic cells may be combined with sunitinib or a similar or related receptor tyrosine kinase inhibitors. Further, also antibodies against PD-1 (Programmed cell death protein 1) or against PD-1 ligands may be combined with the present adenovirus-infected pro-inflammatory dendritic cells. PD-1 is a cell surface receptor known to play an important role in down-regulating the immune system and promoting self tolerance by suppressing T cell inflammatory activity. Antibodies against PD-1 includes e.g. nivolumab, pembrolizumab, and pidilizumab. Further, also antibodies targeting PD-1 ligands, e.g. atezolizumab and avelumab, may find use in supplementing treatment with the present adenovirus-infected pro-inflammatory dendritic cells. Furthermore, the present adenovirus-infected pro-inflammatory dendritic cells may also be combined with e.g. gemcitabine or 5-fluorouracil.

Similarly, an embodiment relates to use of such adenovirus-infected allogeneic pro-inflammatory dendritic cells for use in the manufacture of a medicament for the treatment of cancer.

A further embodiment relates to a method of treating cancer, wherein an adenovirus-infected pro-inflammatory dendritic cell is administrated to a patient in need of such treatment. The administrated dose should be sufficient to activate the patient's own DCs to develop into tumor-loaded migratory DCs. In order to induce a MLR, the administered adenovirus-infected pro-inflammatory dendritic cells are allogeneic to the patient.

The adenovirus-infected pro-inflammatory dendritic cell does typically not expressing any tumor antigen. It is thus preferably administered intra-tumorally. As recognized in the art (Laurell, A et al Intratumorally injected pro-inflammatory allogeneic dendritic cells as immune enhancers: a first-in-human study in unfavourable risk patients with metastatic renal cell carcinoma. Journal for ImmunoTherapy of Cancer, 2017, 5: 52), administration allogeneic pro-inflammatory dendritic cells intra-tumorally induce activation of tumor-specific T cells and intratumoral infiltration of CD8+ T cells. The infected pro-inflammatory dendritic cells that have also been transduced with genes encoding tumor antigens can be administered intratumorally, but are preferably administered into normal dermal or subcuatenous tissues or into lymph nodes.

A further embodiment relates to a method of providing such a pro-inflammatory dendritic cells as disclosed herein, i.e. pro-inflammatory dendritic cells having improved ability to activate allogeneic T-cells through the direct pathway of allorecognition, and being infected with an adenovirus. This method comprises the steps of:
- providing immature dendritic cells;
- infecting the dendritic cells with an adenovirus; and
- maturating the immature dendritic cells into a pro-inflammatory dendritic cell, wherein said maturation is performed by addition of the Toll-like receptor 3 (TLR3)-ligand poly-I:C, a TLR7/8-ligand, such as Resiquimod, and interferon gamma (IFN-γ) to induce maturation of the immature dendritic cell.

Further aspects in relation to the provision of adenovirus infected dendritic cells have already been described herein and are equally applicable to this embodiment.

Without further elaboration, it is believed that one skilled in the art can, using the preceding description and the following experimental part, utilize the present invention to its fullest extent. The preferred specific embodiments described herein are, therefore, to be construed as merely illustrative and not limitative of the remainder of the description in any way whatsoever. Further, although the present invention has been described above with reference to specific embodiments, it is not intended to be limited to the specific form set forth herein. Rather, the invention is limited only by the accompanying claims and, other embodiments than the specific above are equally possible within the scope of these appended claims, e.g. different than those described above.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous.

In addition, singular references do not exclude a plurality. The terms "a", "an", "first", "second" etc do not preclude a plurality.

### BRIEF DESCRIPTION OF THE DRAWINGS

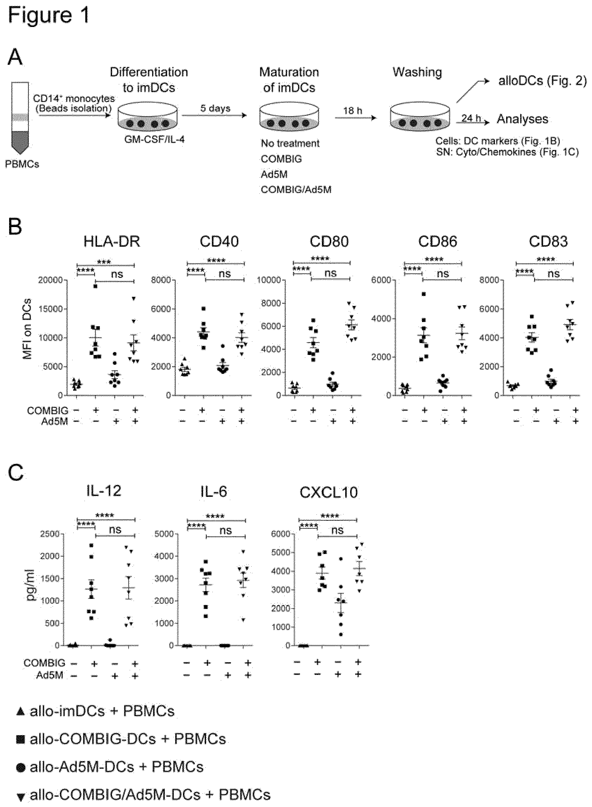

**Figure 1****. COMBIG/Ad5M-matured DCs express a mature phenotype and Th1-polarized cytokine secretion profile.** (Data are shown as mean±SEM (n.s. p≥0.05; * P<0.05; ** P<0.01; *** P<0.001; **** P<0.0001)).
   **(A)** CD14⁺ monocytes were isolated from healthy donor PBMCs, differentiated into imDCs by GM-CSF/IL-4 for 5 days, matured under different conditions for 18h, washed and further cultured for 24h and analyzed.
   **(B)** DCs were characterized for HLA-DR, CD40, CD80, CD86 and CD83 expression by flow cytometry. Mean fluorescence intensity (MFI) for each marker on DCs (CD14⁻CD1a⁺) produced from eight donors are shown.
   **(C)** ELISA was used to measure IL-12p70, IL-6 and CXCL10 secretion for each PBMC donor.

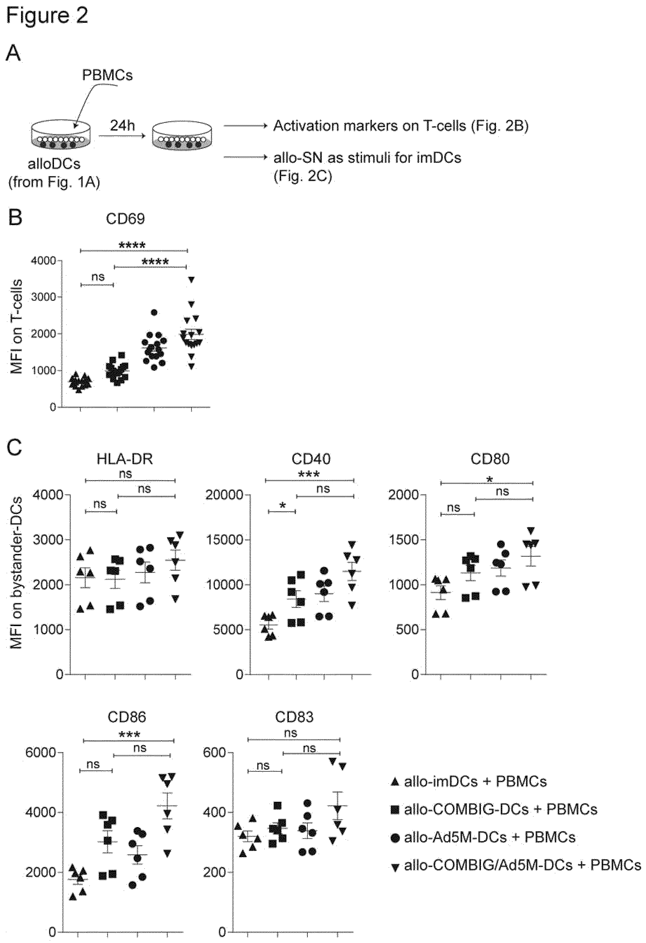

**Figure 2****. Allo-COMBIG/Ad5M-DCs activate innate and adaptive immune cells** ***in** vitro.* (Data are shown as mean±SEM (n.s. p≥0.05; * P<0.05; ** P<0.01; *** P<0.001; **** P<0.0001))
   **(A)** After maturation for 18h and washing (samples as described in Figure 1A) the DCs, here called alloDCs, were co-cultured with allogeneic PBMCs (meaning from a different donor). After 24h of co-culture, activation of T cells in the PBMC pool was characterized by the expression of CD69.
   **(B)** Mean fluorescence intensities for CD69 on T-cells (CD3⁺CD56⁻) from sixteen individual combinations of eight unrelated donors are shown.
   **(C)** The MLR (mixed leukocyte reaction) supernatants (allo-SN) from the alloDC-PBMC co-cultures were used as maturation stimuli for bystander immature (im) DCs (DCs from the same donor as the PBMCs) to mimic a scenario for host "bystander" imDCs. Bystander DC maturation was assessed by the upregulation of HLA-DR, CD40, CD80, CD86 and CD83 as shown in MFI scatter plots for each marker on bystander-DCs (CD14⁻CD1a⁺). Six individual combinations from three unrelated donors were evaluated.

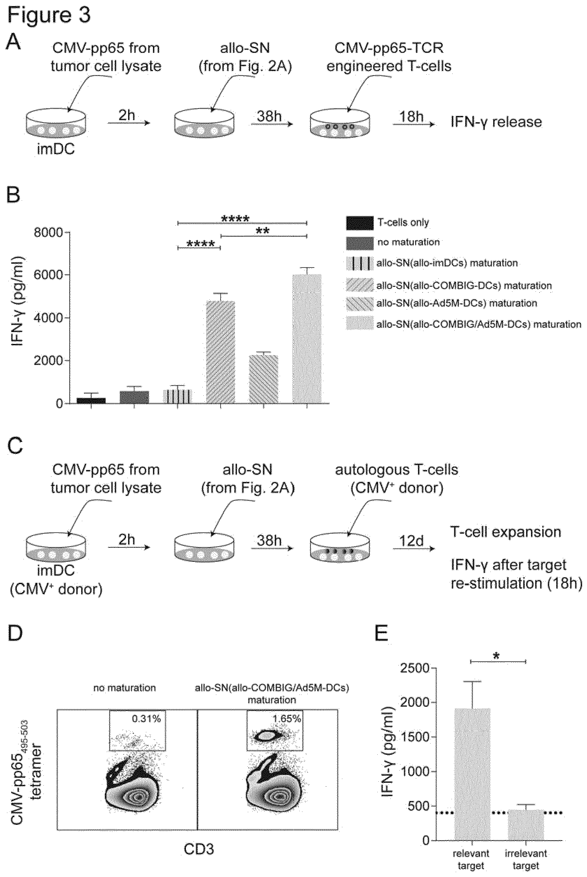

**Figure 3****. Activation and expansion of antigen-specific T-cells induced by cross-presentation of the antigen by autologous bystander-DCs matured by the MLR supernatant (allo-SN) from PMBC allo-COMBIG/Ad5M-DC co-cultures.**
   **(A)** Bystander im DCs from HLA-A2⁺ donors (bystander-DCs) were pulsed for 2h with lysate from tumor cells expressing the CMV-pp65 antigen (A549(pp65)) and matured for 38h by allo-SN from the various co-cultures (Figure 2A). The cross-presenting bystander-DCs were then mixed for 18h with autologous T-cells, engineered with a TCR against the HLA-A2-restricted CMV-pp65₄₉₅₋₅₀₃ peptide.
   **(B)** ELISA was used to measure the concentration of IFN-γ released by the engineered antigen-specific T-cells into the supernatants. Six individual combinations from three unrelated donors were examined.
   **(C)** Bystander-DCs from CMV-seropositive, HLA-A2⁺ donor were prepared as mentioned above and used to stimulate autologous (with respect to the bystander DCs), non-engineered, T cells for 12 days in the presence of low dose IL-2 (20IU/ml).
   **(D)** CMV-pp65-specific expansion of non-engineered T cells was quantified by flow cytometry using PE-conjugated HLA-A*0201/pp65₄₉₅₋₅₀₃ tetramer. FACS plots from one representative individual combination out of four using the allo-SN from PBMC allo-COMBIG/Ad5M-DC co-cultures as maturation stimuli are shown.
   **(E)** The expanded non-engineered T-cells were then re-stimulated by exposure to T2 cells loaded either with CMV-pp65₄₉₅₋₅₀₃ peptide (relevant target) or TARP₄₋₁₃ peptide (irrelevant target). ELISA was used to measure the concentration of IFN-γ in supernatants harvested 18h after re-stimulation. The dotted line indicates the background IFN-γ release from T-cells stimulated by immature bystander-DCs. Data are shown as mean±SEM (* P<0.05; ** P<0.01; **** P<0.0001).

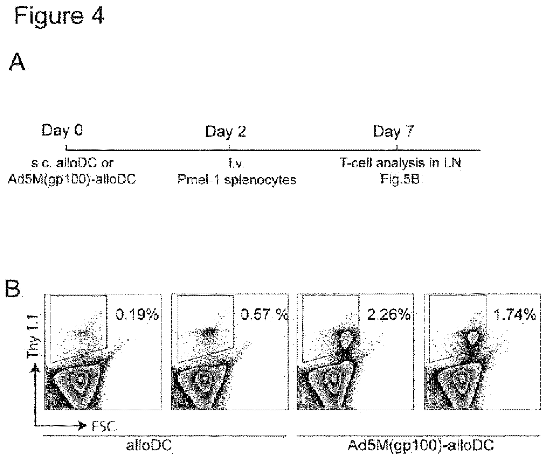

**Figure 4****. Prophylactic vaccination with COMBIG-DC transduced with the Ad5M vector coding for gp-100 indirectly prime gp-100 specific T cells**
   **(A)** The ability of COMBIG DCs (alloDCs) transduced with the Ad5M vector coding for the tumor antigen pg100 (Ad5M(gp100)-alloDCs) to indirectly stimulate gp100-specific T-cells in non-tumor-bearing mice was assessed by combining s.c. injection of Ad5M(gp100)-alloDCs (day 0) with intravenous (i.v.) adoptive cell transfer of gp100-specific Thy1.1⁺ pmel-1 splenocytes (day 2). T-cell read out was performed five days later by Thy1.1 staining (day 7).
   **(B)** Representative scatter plots of the specific proliferation of Thy1.1⁺ pmel-1 T-cells (Thy1.1⁺) in response to alloDCs or Ad5M(gp100)-alloDCs from two mice are shown.

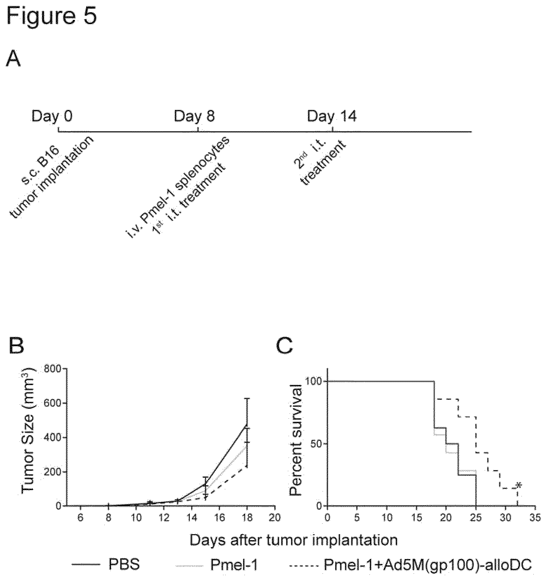

**Figure 5****. Therapeutic vaccination with COMBIG-DC transduced with the Ad5M vector coding for gp-100 decreases tumor growth and prolongs survival**
   **(A)** In a therapeutic setting, C57BL/6NRj mice were first injected s.c. into the hind flank with 1×10⁵ B16-F10 cells (day 0) and received 1×10⁷ pmel-1 splenocytes i.v. (day 8). The treatment was combined with i.t. injections of 1×10⁶ Ad5M(gp100)-alloDCs (day 8 and 14) or PBS as negative control. Tumor growth was monitored by caliper measurement.
   **(B)** Average tumor growth is presented for each treatment (n=8 per group).
   **(C)** Mice survival is shown by the Kaplan-Meier survival curve and compared by log-rank test (n=8 per group) (* P<0.05).

### EXPERIMENTAL

The following examples are mere examples and should by no mean be interpreted to limit the scope of the invention. Rather, the invention is limited only by the accompanying claims.

### EXAMPLE 1 - COMBIG-DCs transduced with an adenovirus vector without any transgene

In example 1, the impact of infecting immature DCs with an empty adenoviral vector (Ad5M) on activation/maturation induced by the Combig-cocktail (Poly-I:C, R848 and IFN-gamma) alone was evaluated (e.g. phenotypic maturation and secretion of inflammatory factors, e.g. IL-6, IL-12 and CXCL10, were studied). In brief, it was seen that Ad5M infection does not impair the ability of the Combig-cocktail to induce maturation of pro-inflammatory DCs (cf. Fig. 1). These data are in line with the prevailing opinion. However, in contrast to prevailing opinion it was found that infection with Ad5M improves, and not impairs, as presumed (cf. e.g. Jonuleit et al in Gene Therapy (2000) 7, 249-254; Tan et al in BLOOD, (2005), 105(10), 3824- 3832; and Newton et al in Immunology, 125, 469-479), the ability of Combig-DCs to stimulate the direct pathway of allorecognition, as measured by the activation (CD69 expression) of allogeneic T cells during a mixed leukocyte reaction (MLR) (cf. Fig. 2B)

Moreover, the finding that activation/maturation of bystander-DCs with MLR-supernatants from co-cultures of allo COMBIG-Ad5M DCs and PBMCs was increased (Fig. 2C) and in turn provides improved capacity of bystander DCs to activate autologous antigen-specific T cells producing the Th1 associated cytokine IFN-gamma (cf. Fig. 3B and 3D) implies that COMBIG-DCs transduced with the Ad5M vector are useful as an improved cell-based allogeneic immune enhancer in which the mode of action is crucially dependent on the direct pathway of allorecognition.

### Materials and Methods

### Cell lines

K562(Luc), A549(pp65) and T2 cells (ATCC) were cultured in RPMI-1640 supplemented with 10% heat-inactivated FBS, 1% PeSt and 1% HEPES. All components and culture media were from Thermo Fisher Scientific. K562(Luc) cells were engineered with a lentivirus vector to express firefly luciferase and A549(pp65) cells to express the cytomegalovirus (CMV)-pp65 antigen. All cells were cultured in a humidified incubator with a 5% CO₂ atmosphere at 37°C.

### Production of the recombinant virus

The Ad5M vector was constructed and produced as previously described (Yu, D., C. Jin, J. Leja, N. Majdalani, B. Nilsson, F. Eriksson, and M. Essand. 2011. Adenovirus with hexon Tat-protein transduction domain modification exhibits increased therapeutic effect in experimental neuroblastoma and neuroendocrine tumors. J Virol 85: 13114-13123). Ad5M is an E1-deleted human adenovirus serotype-5 (Ad5) vector with fiber shaft and knob from serotype-35 and a hexon modification to enhance transduction efficacy (Yu, D. et al 2013. Adenovirus serotype 5 vectors with Tat-PTD modified hexon and serotype 35 fiber show greatly enhanced transduction capacity of primary cell cultures. PLoS One 8: e54952). Ad5M does not encode any transgene. Titers were determined by quantitative PCR as encapsidated virus genomes (evg) per ml (Yu, D. et al 2013. Adenovirus serotype 5 vectors with Tat-PTD modified hexon and serotype 35 fiber show greatly enhanced transduction capacity of primary cell cultures. PLoS One 8: e54952).

### Isolation of human PBMCs and generation of DCs

Buffy coats from healthy donors were obtained from the blood bank at the Uppsala University Hospital, Uppsala, Sweden. PBMCs were isolated by Ficoll-Paque Premium separation (GE Healthcare Life Science) and cultured in RPMI-1640 supplemented with 10% heat-inactivated FBS, 1%PeSt, 0.5%L-glutamine, 1%HEPES and 20mM β-mercaptoethanol (DC medium).

### Generation and treatment of DCs.

Monocytes isolated from PBMCs by CD14⁺ positive magnetic selection (Miltenyi Biotec) were differentiated to immature DCs (imDCs) using 20ng/mL human IL-4 and 100ng/mL GM-CSF (Gentaur) for 5 days. Medium was replaced every 2 days. On day 5, cells were either left untreated (imDCs) or matured for 18h with a cocktail of maturation stimuli (Napolitani, G.. et al. 2005. Selected Toll-like receptor agonist combinations synergistically trigger a T helper type 1-polarizing program in dendritic cells. Nat Immunol 6: 769-776; Lovgren, T. et al 2017. Enhanced stimulation of human tumor-specific T cells by dendritic cells matured in the presence of interferon-gamma and multiple toll-like receptor agonists. Cancer Immunol Immunother) consisting of 2.5µg/mL R848 (InvivoGen), 20µg/mL polyinosinic:polycytidylic acid (polyI:C) (Sigma-Aldrich) and 1000IU/mL IFN-γ (Shenandoah Biotechnology). This maturation cocktail was named "Combined Toll-like receptor ligands with IFN-γ" (COMBIG). DCs matured with COMBIG used as allogeneic stimuli are referred to as allo-COMBIG-DCs. Immature DCs treated with Ad5M (2000 evg/cell) for 18h are referred to as allo-Ad5M-DCs while allo-COMBIG/Ad5M-DCs were treated with both COMBIG and Ad5M for 18h. After washing, cells were further cultured in fresh DC medium.

### Human Cytokine array and Cytokine release assay

Supernatants from DC cultures (2.5-5x10⁵ cells/well in 48-well plates) were collected. ELISA kits were used to study the production of IL-12p70 (Mabtech), IL-6 (BioLegend) and CXCL10 (BioLegend).

### Flow cytometry

*Human DC phenotyping.* Anti-CD1a-BV510 (BD Biosciences), anti-CD14-APC/Cy7, anti-HLA-DR-perCP (MHC class II), anti-CD40-FITC, anti-CD80-PE, anti-CD83-APC and anti-CD86-BV421 were used to evaluate the DCs.

### Activation of T-cells in allogeneic mixed leukocyte reaction.

AlloDCs were co-cultured with PBMCs from unrelated donors (MLR) at ratio 1:5 (alloDCs:PBMCs). Activation was assessed 24h later by flow cytometry with anti-CD3-FITC and anti-CD69-BV510. ELISA was used for the detection of IFN-γ (Mabtech) in the allogeneic co-culture supernatant (allo-SN).

### Activation of bystander immature DCs.

AlloDCs were co-cultured with PBMCs at ratio 1:5 (alloDCs:PBMCs) for 24h and the allo-SN was used as a maturation stimuli for immature bystander-DCs. Bystander-DC maturation was assessed 48h later by flow cytometry as before.

All antibodies were purchased from BioLegend, unless specified elsewhere. Data acquisition was performed using a FACSCanto II (BD Biosciences) flow cytometer, and the analysis was performed using FlowJo software (version 7.6.5; Tree Star).

### DC antigen cross-presentation and T-cell stimulation assays

### Cross-presentation of CMV-pp65 by DCs for specific stimulation of autologous CMV-pp65₄₉₅₋₅₀₃ TCR-modified T-cells.

Immature DCs (HLA-A2⁺) were cultured for 2h at 37°C with freeze/thawed cell-lysate from A549(pp65) tumor cells, as a mean of providing CMV-pp65 protein to DCs exogenously. The DCs were subsequently matured in supernatant from alloDC/PBMCs (allo-SNs) for 38h. Autologous T-cells engineered to express an HLA-A*0201-restricted T-cell receptor (TCR) for the CMV-pp65₄₉₅₋₅₀₃ epitope (Hillerdal, V., et al 2016. Avidity characterization of genetically engineered T-cells with novel and established approaches. BMC Immunol 17: 23) were then mixed with CMV-pp65 cross-presenting DCs at ratio 5:1 (T-cells:DCs) and cultured in fresh medium for 18h. TCR-specific T-cell activation was assessed by the secretion of IFN-γ.

### Expansion and re-stimulation of T-cells by cross-presenting DCs.

T-cells (non-modified) from CMV-seropositive, HLA-A2⁺ donors were mixed with autologous CMV-pp65 cross-presenting DCs prepared as above at ratio of 20:1 (T-cells:DCs) and cultured in fresh medium for a total of 12 days. Medium with 30IU/ml IL-2 (Proleukin, Novartis) and 20ng/ml IL-7 (Nordic Biosite) was replaced after 7 days. CMV-pp65-specific T-cell expansion was detected with an HLA-A*0201/pp65₄₉₅₋₅₀₃ tetramer (Beckman Coulter).

T2 cells (HLA-A2⁺) were pulsed with 5µg/ml CMV-pp65₄₉₅₋₅₀₃ peptide or the HLA-A2 binding irrelevant TARP(P5L)₄₋₁₃ peptide. The pulsed T2 cells were used to re-stimulate the expanded CMV-pp65-specific T-cells for 18h. IFN-γ release was measured as an indicator of T-cell activation.

### Statistics

The data are reported as mean±SEM. Statistical analysis was performed by GraphPad prism software version 6.01 (La Jolla). Statistical analyses were performed using parametric One-way ANOVA with Holm-Sidak test for multiple comparison correction. Student t-test was used when only two groups were evaluated. Values with P<0.05 were considered to be statistically significant.

### Results and Discussion

### COMBIG-matured and COMBIG/Ad5M-matured DCs exhibit a phenotype and cytokine secretion profile associated with T-helper-cell (Th1) polarization

The maturation status of DCs is very crucial for the induction of optimal effector responses in autologous DC cancer vaccination (Sabado, R. L., and N. Bhardwaj. 2010. Directing dendritic cell immunotherapy towards successful cancer treatment. Immunotherapy 2: 37-56.). Interestingly, it has also been found important for the activation of allogeneic T-cells (Jonuleit, H. et al 2000. Induction of interleukin 10-producing, nonproliferating CD4(+) T cells with regulatory properties by repetitive stimulation with allogeneic immature human dendritic cells. J Exp Med 192: 1213-1222.). In our experiments, immature DCs (imDCs) were left untreated or matured for 18h by COMBIG, Ad5M or their combination. The use of the infection-enhanced adenoviral vector Ad5M can facilitate loading of DCs with tumor antigens, a standard *ex vivo* practice to modify patient-derived DCs (Tacken, P. J., and C. G. Figdor. 2011. Targeted antigen delivery and activation of dendritic cells in vivo: steps towards cost effective vaccines. Semin Immunol 23: 12-20.). For the sake of having a simple system to assess the maturation effect of Ad5M on DCs, in this study Ad5M was used without a transgene. Cells were washed and cultured for another 24h in fresh DC medium without addition of any cytokines. DC phenotypic changes were assessed by flow cytometry and the supernatants (SN) were collected to map the secretion profiles (Fig. 1A).

COMBIG maturation, alone or combined with Ad5M, induced upregulation of HLA-DR, CD40, CD80, CD86 and CD83, implying a mature and activated phenotype (Fig. 1B). COMBIG-matured and COMBIG/Ad5M-matured DCs demonstrated also elevated secretion of pro-inflammatory cytokines and chemokines. Thus, confirming the transduction didn't impair the maturation of imDCs into pro-inflammatory DCs.

Taken together, our data indicate that Ad5M/COMBIG-maturation is well tolerated by human monocyte-derived DCs and resulted in the generation of a fully matured and pro-inflammatory DC phenotype.

### DCs matured with the COMBIG-cocktail combined with Ad5M infection increase their capacity to activate allogeneic T-cells

T-cell activation in the allogeneic PBMC pool by co-cultured Combig-DCs or COMBIG/Ad5M DCs was assessed by the upregulation of the early activation protein CD69. Interestingly, in marked contrast to prevailing opinion T-cell activation was higher in co-cultures with allo-COMBIG infected with the Ad5M virus vector compared to those of non-infected allo-COMBIC-DCs.

### Supernatants from co-cultures of allo-COMBIG/Ad5M-DCs and PBMCs induce an efficient maturation bystander-DCs

MLR supernatants (Allo-SN) from co-cultures of PBMCs with allo-COMBIG-DCs or allo-COMBIG/Ad5M-DCs was found to significantly induce upregulation of CD40 on immature bystander-DCs. In line with the increased capacity of allo COMBIG/Ad5 DCs to activate allogeneic T cells, MLR supernatants from PBMC allo-COMBIG/Ad5M-DC co-cultures, but not MLR supernatants from PBMC non-infected allo-COMBIG-DC co-cultures, additionally led to significant upregulation of the maturation markers CD80 and CD86 on the bystander-DCs (Fig. 2E).

### Bystander-DCs, matured by the MLR-supernatant from PMBC allo-COMBIG/Ad5M-DC co-cultures, efficiently cross-present antigens for T-cell expansion.

Beside DC maturation, antigen-uptake and antigen-presentation play important roles in inducing adaptive tumor immunity (Vandenberk, L. et al 2015. Exploiting the Immunogenic Potential of Cancer Cells for Improved Dendritic Cell Vaccines. Front Immunol 6: 663).

In order to test antigen-uptake and antigen-presentation we used an *in vitro* model system where DCs from HLA-A2⁺ donors (bystander-DCs) were incubated with cell lysate from tumor cells (A549(pp65)) expressing the full length CMV-pp65 protein, mimicking the condition of lysed tumor cells (Fig. 3A, C). These antigen-loaded immature bystander-DCs were then matured with MLR supernatants from PBMC-alloDC co-cultures. The exogenous pp65 antigen needs to be processed by the bystander-DCs into the pp65₄₉₅₋₅₀₃ peptide and cross-presented on HLA-A2 (MHC class I) in order to stimulate autologous T-cells, engineered with a TCR specific for CMV-pp65₄₉₅₋₅₀₃. We found that antigen-loaded bystander-DCs matured in MLR supernatants could efficiently cross-present the antigen and stimulate engineered CMV-pp65₄₉₅₋₅₀₃ TCR-specific T-cells (Fig 3A) to secrete high amounts of IFN-γ, with MLR supernatants from PBMC allo-COMBIG/Ad5M-DC co-cultures yielding significantly better activation compared to the MLR supernatants from PBMCs co-cultured with untransduced allo-COMBIG-DC (Fig. 3B).

We next wanted to test whether endogenous CMV-pp65-specific T-cells (non-engineered) could be activated and expanded. In this case we started with bystander-DCs from HLA-A2⁺ CMV-seropositive donors, loaded with pp65-containing tumor cell lysate and matured with allo-SN from PBMC allo-COMBIG/Ad5M-DC co-cultures (Fig. 3C). These bystander-DCs yielded a 5-fold expansion of autologous CMV-pp65-specific T-cells from CMV-seropositive donors (Fig. 3D). The ability of those T-cells to recognize their target and exert effector functions was tested in a re-stimulation assay. The expanded T-cells were exposed to T2 cells pulsed with either the HLA-A2-restricted pp65₄₉₅₋₅₀₃ peptide or an irrelevant the HLA-A2-restricted peptide and the IFN-γ release was measured. Upon re-stimulation with the pp65-positive target cells, high IFN-γ release could be detected from the T-cells expanded by pp65-presenting bystander-DCs matured with allo-SN from PBMC allo-COMBIG/Ad5M-DCs co-cultures (Fig. 3E). The expanded T-cells were also confirmed as antigen-specific since exposure to T2 cells pulsed with an irrelevant peptide led only to background amounts of IFN-γ release (Fig. 3E).

In summary, we postulate a rationale for using allo COMBIG DCs infected with the adenovirus Ad5M (allo COMBIG/Ad5M DCs) as an immune enhancer inducing bystander DC maturation and subsequent priming of antigen-specific T cells. When compared to non-infected COMBIG-DCs, COMBIG-DCs infected with the Ad5M virus (COMBIG/Ad5M DCs) were surprisingly found to create a pro-inflammatory milieu with enhanced capacity to induce bystander-DC maturation when co-cultured with allogeneic PBMCs. When a model antigen was provided exogenously, mimicking tumor cell lysate, bystander DCs matured with MLR supernatants from co-cultures of allo COMBIG/Ad5M DCs and PBMCs could efficiently digest and cross-present the model antigen and provoke antigen-specific cytotoxic T-cell expansion and activation.

### EXAMPLE 2 - COMBIG DC:s infected and transduced with adenovirus vector coding for a tumor associated antigen (glycoprotein 100)

In example 2, alloDCs infected with the Ad5M virus with genes encoding for the tumor antigen gp100 were evaluated. It was found that subcutaneous administration of Ad5M(gp100)-alloDCs stimulated the proliferation of adoptively transferred pmel-1 (gp-100-directed) CD8+ T-cells in the prophylactic setting. Moreover intratumoral administration of Ad5M(gp100)-alloDCs in the therapeutic setting resulted in delayed tumor growth and prolonged survival.

### Materials and Methods

### Production of the recombinant virus

The Ad5M(MOCK) and Ad5M(gp100) vectors were constructed and produced as previously described (Yu D, et al. Adenovirus with hexon Tat-protein transduction domain modification exhibits increased therapeutic effect in experimental neuroblastoma and neuroendocrine tumors. J Virol 2011;85(24):13114-23 doi 10.1128/JVI.05759-11). They are E1 deleted adenovirus serotype 5 (Ad5) vectors having the fiber shaft and knob replaced with the serotype-35 counterpart. They also contain a cell-penetrating peptide from HIV Tat in the hypervariable loop of their hexon proteins (major capsid protein). They are infection-enhanced vectors with improved ability to transduce most hematopoietic cells including DCs (Yu D, et al. Adenovirus serotype 5 vectors with Tat-PTD modified hexon and serotype 35 fiber show greatly enhanced transduction capacity of primary cell cultures. PLoS One 2013;8(1):e54952 doi 10.1371/journal.pone.0054952). Ad5M(MOCK) does not encode any transgene, while Ad5M(gp100) encodes the human melanoma antigen glycoprotein 100 (gp100, amino acids 25-33). Titers were determined by quantitative polymerase chain reaction as encapsidated virus genomes (evg) per ml (Yu D, et al. Adenovirus serotype 5 vectors with Tat-PTD modified hexon and serotype 35 fiber show greatly enhanced transduction capacity of primary cell cultures. PLoS One 2013;8(1):e54952 doi 10.1371/journal.pone.0054952).

### Cell lines

The 911 cell line (Crucell, Leiden, The Netherlands) used for adenovirus production was cultured in DMEM supplemented with 10% heat inactivated FBS, 1% penicillin/streptomycin (PeSt) and 1% sodium pyruvate. The mouse melanoma cell line B16-F10 (ATCC, Rockville, MD) were cultured in DMEM supplemented with 10% heat inactivated FBS, 1% PeSt and 1% sodium pyruvate. The mouse melanoma cell lines Hcmel3 and Hcmel12 are derived from a primary HGF-CDK4^{(R24C)} melanoma model (Bald T, et al. Ultraviolet-radiation-induced inflammation promotes angiotropism and metastasis in melanoma. Nature 2014;507(7490):109-13; Landsberg J, et al. Melanomas resist T-cell therapy through inflammation-induced reversible dedifferentiation. Nature 2012;490(7420):412-6) and are a kind gift from Prof. Thomas Tüting, University Hospital Magdeburg. Both Hcmel3 and Hcmel12 were cultured in RPMI-1640 supplemented with 10% heat inactivated FBS. All components and culture media were from Thermo Fisher Scientific (Carlsbad, CA). All cells were cultured in a humidified incubator with a 5% CO₂ atmosphere at 37°C.

### Isolation and maturation of mouse bone marrow-derived allogeneic DCs

Bone marrow-derived DCs were generated from the femur and tibia of female 7-8 week old wild-type (wt) BALB/c mice (H-2D^{d}) (The Janvier Labs, France) by exposing the bone marrow and flushing out the cells with a sterile syringe. The harvested cells were cultured in IMDM supplemented with 10% heat inactivated FBS, 1% PeSt, 1% HEPES, 50 µM β-mercaptoethanol. Culture medium was supplemented with 20ng/mL recombinant murine IL-4 and 20ng/mL recombinant murine GM-CSF (Nordic BioSite, Stockholm, Sweden). Bone marrow cells were plated on non-treated Petri dishes (Sarstedt, Nümbrecht, Germany). Medium was replaced every 3 days. On day 7 the non-adherent imDCs were harvested and treated for 18h with a cocktail of combined Toll-like receptor ligands with IFN-γ (COMBIG), consisting of 2.5 µg/mL R848 (InvivoGen, Sam Diego, CA), 20 µg/mL polyinosinic:polycytidylic acid (polyI:C) (Sigma-Aldrich, St. Louis, MO) and 1000 IU/mL IFN-γ (Nordic Biosite) (Napolitani G, Rinaldi A, Bertoni F, Sallusto F, Lanzavecchia A. Selected Toll-like receptor agonist combinations synergistically trigger a T helper type 1-polarizing program in dendritic cells. Nat Immunol 2005;6(8):769-76 doi 10.1038/ni1223) in order to obtain mature alloDCs. Ad5M(gp100)-alloDCs were obtained by transducing pelleted imDCs for 2h, at 37°C with 2000 evg/cell Ad5M(gp100) and matured as above. Cells were cultured in a humidified incubator with a 5% CO₂ atmosphere at 37°C.

### Murine immune cell phenotyping

Flow cytometry was used to phenotype murine T-cells using anti-CD3-PB, anti-CD8a-APC, anti-Thy1.1-PE, anti-Vβ-13-FITC, H-2D^{b}/hgp100₂₅₋₃₃-PE tetramer (Beckman Coulter, San Diego, CA). All antibodies were purchased from BioLegend (San Diego, CA). Data acquisition was performed using a FACSCanto II (BD Biosciences) flow cytometer, and the analysis was performed using FlowJo software (version 7.6.5; Tree Star, Ashland, OR).

### Animal experiments

The Uppsala Research Animal Ethics Committee (C215/12) and the Northern Stockholm Research Animal Ethics Committee (N170/13, N164/15) have approved the animal studies. All *in vivo* experiments were performed with female 6-7 weeks old C57BL/6NRj (H-2D^{b}) mice receiving vaccinations with treated allogeneic DCs (vaccine cells) generated from the bone marrow of 7-8 weeks old female BALB/c (H-2D^{d}) mice and treated as described above. All mice were obtained from Janvier Labs.

**Adoptive transfer of pmel-1 (gp100-specific) splenocytes.** Mice were injected s.c. with 1x10⁶ vaccine cells. Pmel-1 splenocytes (10x10⁶ per mouse) were injected intravenously (i.v.) 48 hours later. The transgenic pmel-1 mice have a C57BL/6NRj (H-2D^{b}) background and around 20% of their splenocytes are Thy1.1⁺ CD8⁺ T-cells that carry a gp100₂₅₋₃₃-specific TCR. Pmel-1 mice were originally obtained from the Jackson Laboratory (Bar Harbor, Maine, USA) and kept in breeding by our group. Draining (inguinal) lymph nodes were harvested 7 days post pmel-1 adoptive splenocyte transfer and digested into single-cell suspensions.

### In vivo assessment of therapeutic efficacy in a B16 melanoma model after intratumoral injection with alloDCs

Mice were injected s.c. into the hind flank with 1×10⁵ B16-F10 tumor cells. For the pmel-1 T-cell transfer experiment, mice received i.v. injection of 1×10⁸ pmel-1 splenocytes on day 8 and i.t. injections of 10⁶ alloDCs on day 8 and 14 post B16-F10 tumor cell inoculation. Tumors were measured with caliper and the tumor size was calculated as the formula: Volume = length × width² × π/6. Mice were sacrificed when the tumor volume exceeded 1 cm³ or if bleeding ulcers developed. Cells were obtained from spleen, blood and lymph node of the sacrificed mice on day 15 post B16-F10 inoculation and stained for expression of the Thy1.1 and Vβ13 pmel-1 T-cell markers.

### Statistics

The data are reported as mean and SEM. Statistical analysis was performed by GraphPad prism software version 6.01 (La Jolla, CA, USA). Statistical analyses were performed using parametric One way ANOVA test (> 2 experimental groups) with Holm-Sidak test for multiple comparison correction and Mann-Whitney U test (only 2 experimental groups). Association of gp100-TCR⁺ T-cells and tumor burden was evaluated by a linear regression model computing Spearman correlation. Statistical comparison of the Kaplan-Meier survival curve was performed using log-rank test. Values with P<0.05 were considered to be statistically significant.

### Results

### Ad5M(gp100)-alloDCs vaccination in a mouse melanoma model augments the proliferation and percistence of adoptively transferred pmel-1 T-cells, delays tumor growth and prolongs survival

We sought to examine if Ad5M(gp100)-alloDCs in combination with adoptive T-cell transfer from pmel-1 splenocytes has a positive survival effect in melanoma-bearing mice. Naive C57BL/6NRj mice were injected s.c on the right hind limb with 1x10⁶ alloDCs or Ad5M(gp100)-alloDCs, followed by intravenous (i.v) adoptive transfer of 10x10⁶ pmel-1 splenocytes (Fig. 4A). In contrast to non-transfected COMBIG-DCs, COMBIG/Ad5M(gp100)-DCs were able to stimulate the proliferation of the adoptively transferred gp100-specific Thy1.1⁺ CD8⁺ T-cells and sustain their presence five days after transfer (Fig. 4B).

As pmel-1 T-cells persist after vaccination with COMBIG/Ad5M(gp100)-DCs, we next evaluated the combinatorial effect of adoptive pmel-1 splenocyte transfer with i.t. injection of COMBIG/Ad5M(gp100)-DCs (Fig. 5A) in a therapeutic setting. The combined treatment delayed tumor growth (Fig. 5B) and significantly prolonged survival of tumor-bearing mice (Fig. 5C).

### Discussion

Taken together the present example demonstrates that the use of COMBIG DCs infected with Ad5M coding for the tumor antigen gp100 can potentiate systemic gp100-specific T-cell responses, suppress tumor growth and prolong survival in a mouse melanoma model. Effective priming of T-cell responses by an immune primer/vaccine requires endogenous DCs to take up antigens, mature and migrate to dLNs where they can productively interact with naive T-cells (Dieu-Nosjean MC, et al Tertiary lymphoid structures in cancer and beyond. Trends Immunol 2014;35(11):571-80; Kleindienst P, et al Endogenous dendritic cells are required for amplification of T cell responses induced by dendritic cell vaccines in vivo. J Immunol 2003;170(6):2817-23), thus indicating that locally injected COMBIG-DC that have been infected with an Ad5M virus coding for a tumor antigens will similar to COBMIG DCs infected with and non-coding Ad5M virus (see Example 1) induce a pro-inflammatory response that stimulate maturation of endogenous bystander DCs and a subsequent efficient priming of tumor-specific T cells.

## Claims

1. A method of providing pro-inflammatory dendritic cells, said pro-inflammatory dendritic cells having improved ability to activate allogeneic T-cells through the direct pathway of allorecognition, and said dendritic cells being infected with an adenovirus, wherein said method comprises the steps of:
- infecting provided dendritic cells with an adenovirus; and
- maturating provided immature dendritic cells into pro-inflammatory dendritic cells,
wherein said maturation is performed by addition of the Toll-like receptor 3 (TLR3)-ligand poly-I:C, a TLR7/8-ligand, and interferon gamma (IFN-γ) to induce maturation of the immature dendritic cell, wherein said pro-inflammatory adenovirus infected dendritic cells have improved ability to activate allogeneic T-cells through the direct pathway of allorecognition compared to corresponding pro-inflammatory dendritic cells not infected with an adenovirus.

2. The method according to claim 1, wherein the TLR7/8-ligand is Resiquimod.

3. The method according to claim 1 or 2, wherein the adenovirus is an adenovirus serotype-5 (Ad5) with fiber shaft and/or knob from adenovirus serotype-35 (Ad35) in place of the Ad5 fiber shaft and/or knob.

4. The method according to claim 1 or 3, wherein the hexon protein of the adenovirus is modified to comprise at least one protein transduction domain (PTD) of the transactivator of transcription (TAT) from human immunodeficiency virus (HIV).

5. The method according to any one of the claims 1 to 4, wherein the adenovirus is an E1-deleted adenovirus.

6. The method according to any one of the claims 1 to 5, wherein the adenovirus does not comprise any gene encoding for a tumor antigen, whereby the pro-inflammatory dendritic cell being infected but not transduced.

7. The method according to any one of the claims 1 to 5, wherein the adenovirus does comprise at least one gene encoding for a tumor associated or tumor-specific antigen, whereby the infected dendritic cell being transduced to express the tumor associated or specific antigen.

8. The method according to claim 7, wherein the tumor antigen is selected from the group consisting of oncoviral antigens, such as human papillomavirus, Epstein-Barr virus, Kaposis sarcoma virus, and Merkel cell polyoma virus, and mutation-derived neoantigens.

9. The method according to any one of the claims 1 to 8, wherein the maturation and the infection with the adenovirus, respectively, are performed simultaneously.

10. The method according to any one of the claims 1 to 9, wherein
- said step of maturating the immature dendritic cell into the pro-inflammatory dendritic cell further comprise the addition of at least one substance selected from the group consisting of TLR2-ligands, TLR4-ligands, TLR9-ligands, Interferon alpha (IFN-α), interleukin 1β (IL-1β), and tumor necrosis factor alpha (TNF-α) to induce activation; and/or
- said step of maturating the immature dendritic cell into the pro-inflammatory dendritic cell does not comprise addition of prostaglandin E2 (PGE2).

11. The method according to any one of claims 1 to 10, wherein the immature dendritic cells are provided by differentiating monocytes into immature dendritic cells by use of interleukin 4 (IL-4) and Granulocyte-Macrophage Colony-Stimulating Factor (GM-CSF), the monocytes having been isolated from peripheral blood leukocytes.

## Patentansprüche

1. Verfahren zum Bereitstellen von proinflammatorischen dendritischen Zellen, wobei die proinflammatorischen dendritischen Zellen verbesserte Fähigkeit aufweisen, allogene T-Zellen durch den direkten Weg der Alloerkennung zu aktivieren, und wobei die dendritischen Zellen mit einem Adenovirus infiziert sind, wobei das Verfahren die Schritte umfasst:
- Infizieren bereitgestellter dendritischer Zellen mit einem Adenovirus; und
- Reifen bereitgestellter dendritischer Zellen zu proinflammatorischen dendritischen Zellen,
wobei die Reifung durch Zugabe des Toll-like-Rezeptor 3 (TLR3)-Liganden Poly-I:C, eines TLR7/8-Liganden und Interferon gamma (IFN-Y) durchgeführt wird, um die Reifung der unreifen dendritischen Zelle zu induzieren, wobei die proinflammatorischen, mit einem Adenovirus infizierten dendritischen Zellen verbesserte Fähigkeit aufweisen, allogene T-Zellen durch den direkten Weg der Alloerkennung zu aktivieren, verglichen zu entsprechenden proinflammatorischen dendritischen Zellen, die nicht mit einem Adenovirus infiziert sind.

2. Verfahren nach Anspruch 1, wobei der TLR7/8-Ligand Resiquimod ist.

3. Verfahren nach Anspruch 1 oder 2, wobei das Adenovirus ein Adenovirus Serotyp-5 (Ad5) mit einem Fiber-Schaft und/oder Kopf vom Adenovirus Serotyp-35 (Ad35) anstelle des Ad5-Fiber-Schaft und/oder Kopfes ist.

4. Verfahren nach Anspruch 1 oder 3, wobei das Hexon-Protein des Adenovirus modifiziert ist, mindestens eine Proteintransduktionsdomäne (PTD) des Transaktivators der Transkription (TAT) aus humanem Immundefizienzvirus (HIV) zu umfassen.

5. Verfahren nach einem beliebigen der Ansprüche 1 bis 4, wobei das Adenovirus ein E1-deletiertes Adenovirus ist.

6. Verfahren nach einem beliebigen der Ansprüche 1 bis 5, wobei das Adenovirus kein Gen zum Kodieren eines Tumorantigens umfasst, wodurch die proinflammatorische dendritische Zelle infiziert, aber nicht transduziert ist.

7. Verfahren nach einem beliebigen der Ansprüche 1 bis 5, wobei das Adenovirus mindestens ein Gen umfasst, das ein tumorassoziiertes oder tumorspezifisches Antigen kodiert, wodurch die infizierte dendritische Zelle transduziert ist, das tumorassoziierte oder tumorspezifische Antigen zu exprimieren.

8. Verfahren nach Anspruch 7, wobei das Tumorantigen aus der Gruppe bestehend aus onkoviralen Antigenen wie humanem Papillomavirus, Eppstein-Barr-Virus, Kaposi-Sarkomvirus und Merkelzell-Polyomavirus und mutationsabgeleiteten Neoantigenen ausgewählt ist.

9. Verfahren nach einem beliebigen der Ansprüche 1 bis 8, wobei die Reifung beziehungsweise die Infektion mit dem Adenovirus gleichzeitig durchgeführt werden.

10. Verfahren nach einem beliebigen der Ansprüche 1 bis 9, wobei
- der Schritt des Reifens der unreifen dendritischen Zelle zur proinflammatorischen dendritischen Zelle des Weiteren die Zugabe von mindestens einer Substanz umfasst, die aus der Gruppe bestehend aus TLR2-Liganden, TLR4-Liganden, TLR9-Liganden, Interferon-alpha (IFN-α), Interleukin 1β (IL-1β) und Tumornekrosefaktor alpha (TNF-α) ausgewählt ist, um Aktivierung zu induzieren; und/oder
- der Schritt des Reifens der unreifen dendritischen Zelle zur proinflammatorischen dendritischen Zelle nicht die Zugabe von Prostaglandin E2 (PGE2) umfasst.

11. Verfahren nach einem beliebigen der Ansprüche 1 bis 10, wobei die unreifen dendritischen Zellen durch Differenzieren von Monozyten zu unreifen dendritischen Zellen durch Verwendung von Interleukin 4 (IL-4) und Granulozyten-Makrophagen-Koloniebildendem Faktor (GM-CSF) bereitgestellt werden, wobei die Monozyten aus peripheren Blutleukozyten isoliert wurden.

## Revendications

1. Procédé pour fournir des cellules dendritiques pro-inflammatoires, lesdites cellules dendritiques pro-inflammatoires présentant une capacité améliorée à activer les lymphocytes T allogéniques par la voie directe d'alloreconnaissance, et lesdites cellules dendritiques étant infectées par un adénovirus, dans lequel ledit procédé comprend les étapes de :
- infection des cellules dendritiques fournies avec un adénovirus ; et
- maturation des cellules dendritiques immatures fournies en cellules dendritiques pro-inflammatoires, dans laquelle ladite maturation est réalisée par ajout du ligand poly-I:C du récepteur de type Toll 3 (TLR3), d'un ligand de TLR7/8, et d'interféron gamma (IFN-γ) pour induire la maturation de la cellule dendritique immature, dans laquelle lesdites cellules dendritiques pro-inflammatoires infectées par un adénovirus présentent une capacité améliorée à activer les lymphocytes T allogéniques par la voie directe d'alloreconnaissance par rapport aux cellules dendritiques pro-inflammatoires correspondantes non infectées par un adénovirus.

2. Procédé selon la revendication 1, dans lequel le ligand de TLR7/8 est le Résiquimod.

3. Procédé selon la revendication 1 ou 2, dans lequel l'adénovirus est un adénovirus de sérotype-5 (Ad5) avec une tige et/ou une tête sphérique de fibre provenant d'un adénovirus de sérotype-35 (Ad35) à la place de la tige et/ou de la tête sphérique de fibre d'Ad5.

4. Procédé selon la revendication 1 ou 3, dans lequel la protéine hexon de l'adénovirus est modifiée pour comprendre au moins un domaine de transduction de protéine (PTD) du transactivateur de transcription (TAT) provenant du virus de l'immunodéficience humaine (VIH).

5. Procédé selon l'une quelconque des revendications 1 à 4, dans lequel l'adénovirus est un adénovirus à E1 délétée.

6. Procédé selon l'une quelconque des revendications 1 à 5, dans lequel l'adénovirus ne comprend aucun gène codant pour un antigène tumoral, grâce à quoi la cellule dendritique pro-inflammatoire est infectée mais non transduite.

7. Procédé selon l'une quelconque des revendications 1 à 5, dans lequel l'adénovirus comprend au moins un gène codant pour un antigène associé à une tumeur ou spécifique d'une tumeur, grâce à quoi la cellule dendritique infectée est transduite pour exprimer l'antigène associé ou spécifique d'une tumeur.

8. Procédé selon la revendication 7, dans lequel l'antigène tumoral est choisi dans le groupe constitué par les antigènes oncoviraux, tels que le papillomavirus humain, le virus d'Epstein-Barr, le virus du sarcome de Kaposis et le virus du polyome à cellules de Merkel, et des néoantigènes dérivés de mutations.

9. Procédé selon l'une quelconque des revendications 1 à 8, dans lequel la maturation et l'infection par l'adénovirus, respectivement, sont effectuées simultanément.

10. Procédé selon l'une quelconque des revendications 1 à 9, dans lequel
- ladite étape de maturation de la cellule dendritique immature en cellule dendritique pro-inflammatoire comprend en outre l'ajout d'au moins une substance choisie dans le groupe constitué par les ligands de TLR2, les ligands de TLR4, les ligands de TLR9, l'interféron alpha (IFN-α), l'interleukine 1β (IL-1β) et le facteur alpha de nécrose tumorale (TNF-α) pour induire l'activation ; et/ou
- ladite étape de maturation de la cellule dendritique immature en cellule dendritique pro-inflammatoire ne comprend pas d'ajout de prostaglandine E2 (PGE2).

11. Procédé selon l'une quelconque des revendications 1 à 10, dans lequel les cellules dendritiques immatures sont obtenues par différenciation de monocytes en cellules dendritiques immatures par l'utilisation d'interleukine 4 (IL-4) et de facteur de stimulation des colonies de granulocytes-macrophages (GM-CSF), les monocytes ayant été isolés à partir de leucocytes du sang périphérique.


### SEQUENCE LISTING

<110> Immunicum AB
<120> IMPROVED ALLOGENEIC DENDRITIC CELLS FOR USE IN CANCER TREATMENT
<130> E89610011
<160> 2
<170> BiSSAP 1.3.6
<210> 1
   <211> 11
   <212> PRT
   <213> Human immunodeficiency virus 1
<400> 1
   Tyr Gly Arg Lys Lys Arg Arg Gln Arg Arg Arg
   1               5                   10
<210> 2
   <211> 27
   <212> PRT
   <213> Artificial Sequence
<220>
   <223> Protein transduction domain with linker
<400> 2
   Ala Gly Gly Gly Ala Gly Gly Gly Tyr Gly Arg Lys Lys Arg Arg Gln
   1               5                   10                  15
   Arg Arg Arg Gly Gly Gly Ala Gly Gly Gly Ala
               20                  25